(19) Europäisches Patentamt
European Patent Office
Office européen des brevets

(11) Veröffentlichungsnummer: **0 387 510 A1**

(12) **EUROPÄISCHE PATENTANMELDUNG**

(21) Anmeldenummer: **90101676.6**

(51) Int. Cl.5: **C07D 215/34, A01N 33/06**

(22) Anmeldetag: **28.01.90**

(30) Priorität: **10.02.89 DE 3903972**

(43) Veröffentlichungstag der Anmeldung:
**19.09.90 Patentblatt 90/38**

(84) Benannte Vertragsstaaten:
**BE CH DE DK FR GB IT LI NL**

(71) Anmelder: **BAYER AG**

**D-5090 Leverkusen 1 Bayerwerk(DE)**

(72) Erfinder: **Krüger, Bernd-Wieland, Dr.**
**Unterboschbach 19**
**D-5060 Bergisch Gladbach 2(DE)**
Erfinder: **König, Klaus, Dr.**
**Zum Hahnenberg 40**
**D-5068 Odenthal(DE)**
Erfinder: **Naumann, Klaus, Dr.**
**Richard-Wagner-Strasse 9**
**D-5090 Leverkusen 1(DE)**
Erfinder: **Brandes, Wilhelm, Dr.**
**Eichendorffstrasse 3**
**D-5653 Leichlingen 1(DE)**
Erfinder: **Dutzmann, Stefan, Dr.**
**Leinenweberweg 33**
**D-4000 Düsseldorf 13(DE)**
Erfinder: **Paulus, Wilfried, Dr.**
**Deswatinesstrasse 90**
**D-4150 Krefeld 1(DE)**

(54) **Substituierte 8-Carbamoyloxychinolin-Derivate, Verfahren zu deren Herstellung, sowie deren Verwendung zur Bekämpfung von Schädlingen.**

(57) Beschrieben wurden 8-Carbamoyloxychinolin-Derivate der Formel

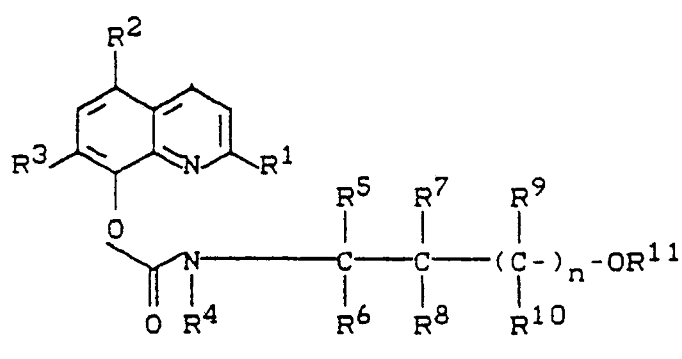

(I)

in welcher
$R^1$ für Wasserstoff oder Methyl steht und
$R^2$ und $R^3$ gleich oder verschieden sind und für Wasserstoff, Halogen, unsubstituiertes oder substituiertes Alkyl oder Alkenyl stehen und
n für null oder eins steht und
$R^{11}$ für unsubstituiertes oder substituiertes Alkyl, $COR^{12}$ oder einen Rest



Xerox Copy Centre

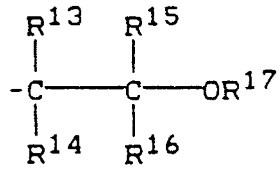

stehen und
$R^4$ bis $R^{10}$ und $R^{13}$ bis $R^{16}$ gleich oder verschieden sind und für Wasserstoff oder unsubstituiertes oder substituiertes Alkyl stehen und
$R^{12}$ und $R^{17}$ für unsubstituiertes oder substituiertes Alkyl stehen, sowie deren Säureadditionssalze,
mit der Maßgabe, daß $R^{11}$ nicht für Alkyl oder einen Rest

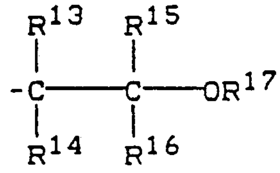

steht, wenn $R^1$ bis $R^4$ für Wasserstoff stehen und n für eins steht,
sowie deren Verwendung zur Bekämpfung von Schädlingen und als Mikrobizide.

Hergestellt werden die 8-Carbamoyloxychinolin-Derivate, indem man z.B. geeignete 8-Hydroxychinoline mit geeigneten Isocyanaten gegebenenfalls in Gegenwart einer geeigneten Base und gegebenenfalls in Gegenwart geeigneter Verdünnungs- oder Lösungsmittel umsetzt.

# Substituierte 8-Carbamoyloxychinolin-Derivate, Verfahren zu deren Herstellung, sowie deren Verwendung zur Bekämpfung von Schädlingen

Die vorliegende Erfindung betrifft neue 8-Carbamoyloxychinolin-Derivate, Verfahren zu ihrer Herstellung und ihre Verwendung zur Bekämpfung von Schädlingen sowie ihre Verwendung als Mikrobizide.

Es ist bereits bekannt, daß bestimmte 8-Alkylaminocarbonyl-oxy-chinoline fungizide Eigenschaften besitzen (vgl. DOS 1 545 797, DOS 1 542 851, FR 1 450 570).

Weiterhin sind viele 8-(Arylaminocarbonyl)-oxychinoline bekannt, die zur Bekämpfung von Schädlingen verwendet werden (vgl. DOS 1 542 851, FR 1 510 067, FR 1 550 499, US 3 384 538).

Weiterhin sind 8-(Heterocyclylcarbonyl)-oxychinoline als Fungizide beschrieben (vgl. EP 254 866).

Bestimmte substituierte 8-Carbamoyloxychinolin-Derivate sind in der Humanmedizin beschrieben (vgl. US 4 530 931, US 4 547 509).

Gegenstand der vorliegenden Anmeldung sind neue 8-Carbamoyloxychinolin-Derivate der allgemeinen Formel (I)

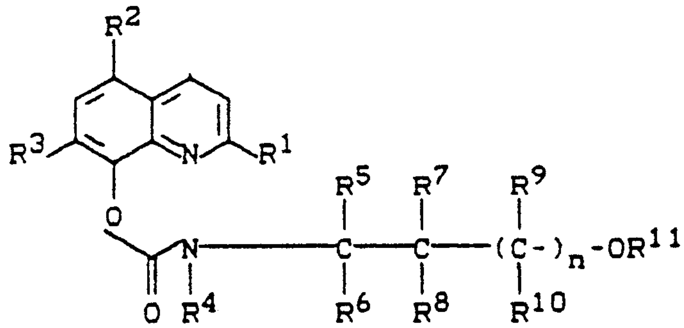

(I)

in welcher

$R^1$ für Wasserstoff oder Methyl steht und

$R^2$ und $R^3$ gleich oder verschieden sind und für Wasserstoff, Halogen, unsubstituiertes oder substituiertes Alkyl oder Alkenyl stehen und

n für null oder eins steht und

$R^{11}$ für unsubstituiertes oder substituiertes Alkyl, $COR^{12}$ oder einen Rest

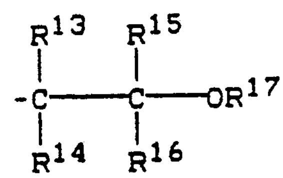

steht und

$R^4$ bis $R^{10}$ und $R^{13}$ bis $R^{16}$ gleich oder verschieden sind und für Wasserstoff oder unsubstituiertes oder substituiertes Alkyl stehen und

$R^{12}$ und $R^{17}$ für unsubstituiertes oder substituiertes Alkyl stehen, sowie deren Säureadditionssalze,

mit der Maßgabe, daß $R^{11}$ nicht für Alkyl oder einen Rest

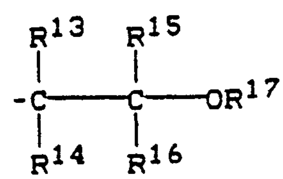

steht, wenn $R^1$ bis $R^4$ für Wasserstoff stehen und n für eins steht.

Die substituierten Chinolinyl-oxy-carbamate der Formel (I) enthalten ein oder mehrere Asymmetriezentren und können somit in Form von Diastereomeren oder Diastereomerengemischen vorliegen, die in unterschiedlichen Mengenverhältnissen anfallen. Vorwiegend fallen sie als Racemate an.

Weiterhin wurde gefunden, daß man die neuen substituierten Chinolinyl-oxy-carbamate der Formel (I)

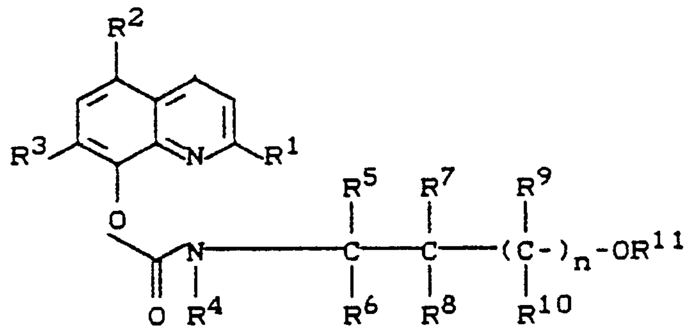

(I)

in welcher

$R^1$ für Wasserstoff oder Methyl steht und

$R^2$ und $R^3$ gleich oder verschieden sind und für Wasserstoff, Halogen, unsubstituiertes oder substituiertes Alkyl oder Alkenyl stehen und

n für null oder eins steht und

$R^{11}$ für unsubstituiertes oder substituiertes Alkyl, $COR^{12}$ oder einen Rest

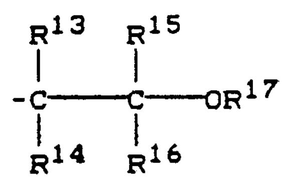

steht und

$R^4$ bis $R^{10}$ und $R^{13}$ bis $R^{16}$ gleich oder verschieden sind und für Wasserstoff oder unsubstituiertes oder substituiertes Alkyl stehen und

$R^{12}$ und $R^{17}$ für unsubstituiertes oder substituiertes Alkyl stehen,

mit der Maßgabe, daß $R^{11}$ nicht für Alkyl oder einen Rest

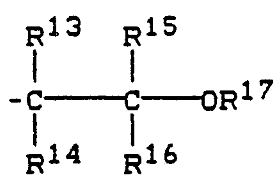

steht, wenn $R^1$ bis $R^4$ für Wasserstoff stehen und n für eins steht,

sowie deren Säureadditionssalze erhält, wenn man 8-Hydroxychinoline der Formel (II)

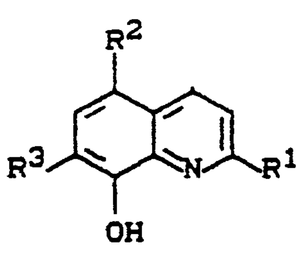

(II)

in welcher

$R^1$, $R^2$ und $R^3$ die obengenannten Bedeutungen haben,

a) mit Isocyanaten der Formel (III)

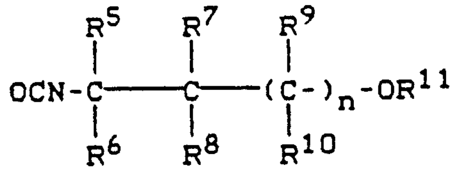

(III)

in welcher
$R^5$ bis $R^{11}$ und n die oben angegebenen Bedeutungen haben,
gegebenenfalls in Gegenwart einer Base und gegebenenfalls in Gegenwart von Verdünnungs- oder Lösungsmitteln umsetzt, oder

b) mit Halogencarbonylverbindungen der Formel (IV)

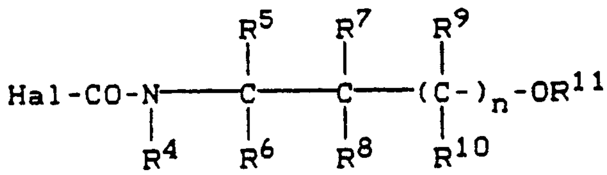

(IV)

in welcher
$R^4$ bis $R^{11}$ und n die oben angegebenen Bedeutungen haben, und
Hal für Halogen, vorzugsweise Chlor steht,
gegebenenfalls in Gegenwart eines Säureakzeptors und gegebenenfalls in Gegenwart eines Lösungs- oder Verdünnungsmittels umsetzt;
und gegebenenfalls anschließend eine Säure addiert.

Schließlich wurde gefunden, daß die substituierten Chinolinyl-oxy-carbamate der Formel (I) eine gute Wirkung gegen Schädlinge besitzen, vor allem eine hohe fungizide Wirksamkeit.

Weiterhin wurde gefunden, daß die erfindungsgemäßen Substanzen ein so breites antimikrobielles Wirkungsspektrum aufweisen, daß sie mit Vorteil als Mikrobizide in mikrobiziden Mitteln zum Schutz technischer Materialien vor Schädigung und Zerstörung durch Mikroorganismen verwendet werden können.

Die neuen Verbindungen können auch mit anderen bekannten, hochwirksamen Verbindungen in synergistischen Mischungen verwendet werden.

Im Rahmen der vorliegenden Erfindung haben die Substituenten vorzugsweise die folgenden Bedeutungen:

Halogen kann, überall wo nicht anders angegeben, Fluor, Chlor, Brom und Iod, bevorzugt Fluor, Chlor und Brom, bedeuten.

Gegebenenfalls substituiertes Alkyl steht im allgemeinen für einen geradkettigen oder verzweigten Kohlenwasserstoffrest mit 1 bis 6 Kohlenstoffatomen, bevorzugt mit 1 bis 4 Kohlenstoffatomen. Beispielhaft seien genannt Methyl, Ethyl, n- und iso-Propyl, n-, sec.-, iso- und tert.-Butyl, Hexyl.

Als Substituent eines Alkylrestes seien beispielsweise Halogen, wie oben definiert, genannt, außerdem Alkoxy und Alkylthio.

Alkoxy bzw. Alkylthio stehen im allgemeinen für einen über Sauerstoff bzw. Schwefel gebundenen, geradkettigen oder verzweigten Kohlenwasserstoffrest mit 1 bis 6, vorzugsweise 1 bis 4 und besonders bevorzugt mit 1 oder 2 Kohlenstoffatomen im Alkoxy- bzw. Alkylthiorest.

Beispielsweise seien für die substituierten Alkylreste genannt: Fluormethyl, Chlormethyl, Brommethyl, Fluorethyl, Chlorethyl, Bromethyl, Fluorpropyl, Chlorpropyl, Brompropyl, Fluorbutyl, Chlorbutyl, Brombutyl, Fluorisopropyl, Chlorisopropyl, Bromisopropyl, Difluormethyl, Trifluormethyl, Dichlormethyl, Trichlormethyl, Difluorethyl, Dichlorethyl, Trifluorethyl, Tetrafluorethyl, Dibrommethyl, Dibromethyl, Dibrompropyl, Dibrombutyl, Difluorbutyl, Dichlorbutyl, Trichlorethyl und Trifluorpropyl. Ganz besonders bevorzugt sind Trifluormethyl, 1-Fluor-2-fluormethyl-prop-(2)-yl und 1-Chlor-2-methyl-prop-(2)-yl. Ferner Methoxymethyl, Methoxyethyl, Methoxypropyl, Methoxybutyl, Methoxypentyl, Ethoxymethyl, Ethoxyethyl, Ethoxypropyl, Ethoxybutyl, Ethoxy pentyl, Propoxymethyl, Propoxyethyl, Propoxypropyl, Propoxybutyl, Propoxypentyl, Butoxymethyl, Butoxyethyl, Butoxypropyl, Butoxybutyl und Butoxypentyl. Besonders bevorzugt ist: 2-Methoxyprop-(2)-yl.

Ferner Methylthiomethyl, Methylthioethyl, Methylthiopropyl, Methylthiobutyl, Methylthiopentyl, Ethylthiomethyl, Ethylthioethyl, Ethylthiopropyl, Ethylthiobutyl, Ethylthiopentyl, Propylthiomethyl, Propylthioethyl,

Propylthiopropyl, Propylthiobutyl, Propylthiopentyl, Butylthiomethyl, Butylthioethyl, Butylthiopropyl.

Alkenyl steht im allgemeinen für einen geradkettigen oder verzweigten Kohlenwasserstoffrest mit 2 bis 6 Kohlenstoffatomen und einer oder mehreren, bevorzugt mit einer oder zwei, Doppelbindungen. Bevorzugt ist Niederalkenyl mit 2 bis 4 Kohlenstoffatomen und einer Doppelbindung. Beispielsweise seien genannt: Vinyl, Allyl, Propenyl, Isopropenyl, Butenyl und Isobutenyl.

Die erfindungsgemäßen substituierten 8-Carbamoyl-oxy-chinolin-Derivate sind durch die Formel (I) allgemein definiert.

Bevorzugt sind Verbindungen der Formel (I), worin

$R^1$ und $R^4$ für Wasserstoff oder Methyl stehen und

$R^2$ und $R^3$ gleich oder verschieden sind und für Wasserstoff, Halogen oder einen geradkettig oder verzweigten, unsubstituierten oder substituierten Alkyl- oder Alkenylrest mit 1 bis 6 bzw. 2 bis 6 Kohlenstoffatomen stehen und

n für null oder eins steht und

$R^{11}$ für geradkettig oder verzweigtes Alkyl mit 1 bis 6 Kohlenstoffatomen, $COR^{12}$ oder einen Rest

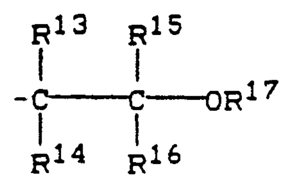

steht und

$R^5$ bis $R^{10}$ und $R^{13}$ bis $R^{16}$ gleich oder verschieden sind und für Wasserstoff oder geradkettig oder verzweigtes Alkyl mit 1 bis 6 Kohlenstoffatomen steht und

$R^{12}$ und $R^{17}$ für geradkettiges oder verzweigtes Alkyl mit 1 bis 6 Kohlenstoffatomen stehen, sowie deren Säureadditionssalze,

mit der Maßgabe, daß $R^{11}$ nicht für Alkyl oder einen Rest

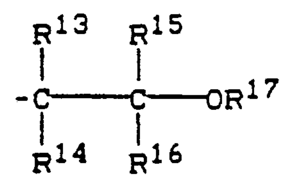

steht, wenn $R^1$ bis $R^4$ für Wasserstoff stehen und n für eins steht.

Besonders bevorzugt sind Verbindungen der Formel (I), worin

$R^1$ und $R^4$ für Wasserstoff oder Methyl stehen und

$R^2$ und $R^3$ gleich oder verschieden sind und für Wasserstoff, Chlor, Brom oder Iod und geradkettig oder verzweigtes Alkyl oder Alkenyl mit 1 bis 4 bzw. 2 bis 4 Kohlenstoffatomen stehen und

n für eins oder insbesondere null steht und

$R^{11}$ für geradkettig oder verzweigtes Alkyl mit 1 bis 4 Kohlenstoffatomen, $COR^{12}$ oder einen Rest

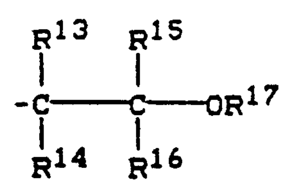

steht und

$R^5$ bis $R^{10}$ und $R^{13}$ bis $R^{16}$ gleich oder verschieden sind und für Wasserstoff oder geradkettig oder verzweigtes Alkyl mit 1 bis 4 Kohlenstoffatomen steht und

$R^{12}$ und $R^{17}$ für geradkettig oder verzweigtes Alkyl mit 1 bis 4 Kohlenstoffatomen stehen, sowie deren Säureadditionssalze,

mit der Maßgabe, daß $R^{11}$ nicht für Alkyl oder einen Rest

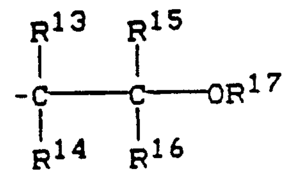

steht, wenn $R^1$ bis $R^4$ für Wasserstoff stehen und n für eins steht.

Ganz besonders bevorzugt sind Verbindungen der Formel (I), worin
$R^1$ und $R^4$ für Wasserstoff stehen und
$R^2$ für Wasserstoff, Chlor oder Brom steht und
$R^3$ für Wasserstoff, Chlor, Brom oder geradkettiges oder verzweigtes Alkenyl mit 2 bis 4 Kohlenstoffatomen steht und
n für null oder eins steht und
$R^{11}$ für geradkettig oder verzweigtes Alkyl mit 1 bis 4 Kohlenstoffatomen, $COR^{12}$ oder einen Rest

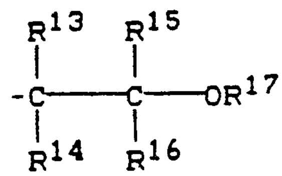

steht und
$R^5$ bis $R^{10}$ und $R^{13}$ bis $R^{16}$ gleich oder verschieden sind und für Wasserstoff oder geradkettig oder verzweigtes Alkyl mit 1 bis 4 Kohlenstoffatomen steht und
$R^{12}$ und $R^{17}$ für geradkettig oder verzweigtes Alkyl mit 1 bis 4 Kohlenstoffatomen stehen, sowie deren Säureadditionssalze,
mit der Maßgabe, daß $R^{11}$ nicht für Alkyl oder einen Rest

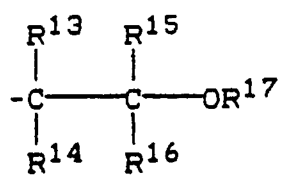

steht, wenn $R^1$ bis $R^4$ für Wasserstoff stehen und n für eins steht.

Insbesondere bevorzugt sind Verbindungen der Formel (I), in denen
$R^2$ für Chlor oder Brom steht und
$R^3$ für Wasserstoff, Chlor oder Brom steht und
n für null steht und
$R^1$ und $R^4$ für Wasserstoff stehen und
$R^5$ bis $R^{17}$ eine der oben angegebenen Bedeutungen hat.

Bevorzugte erfindungsgemäße Verbindungen sind auch Additionsprodukte insbesondere die pflanzenverträglichen Additionsprodukte aus Säuren und denjenigen substituierten Verbindungen der Formel (I), in denen die Substituenten $R^1$ bis $R^{17}$ die Bedeutung haben, die bereits vorzugsweise für diese Substituenten genannt wurden.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie insbesondere Saccharin oder Thiosaccharin.

Verwendet man beispielsweise 5,7-Dibrom-8-hydroxychinolin und Methoxyethylisocyanat als Ausgangsstoffe, so kann der Reaktionsablauf der Verfahrensvariante a) durch das folgende Formelschema wiedergegeben werden:

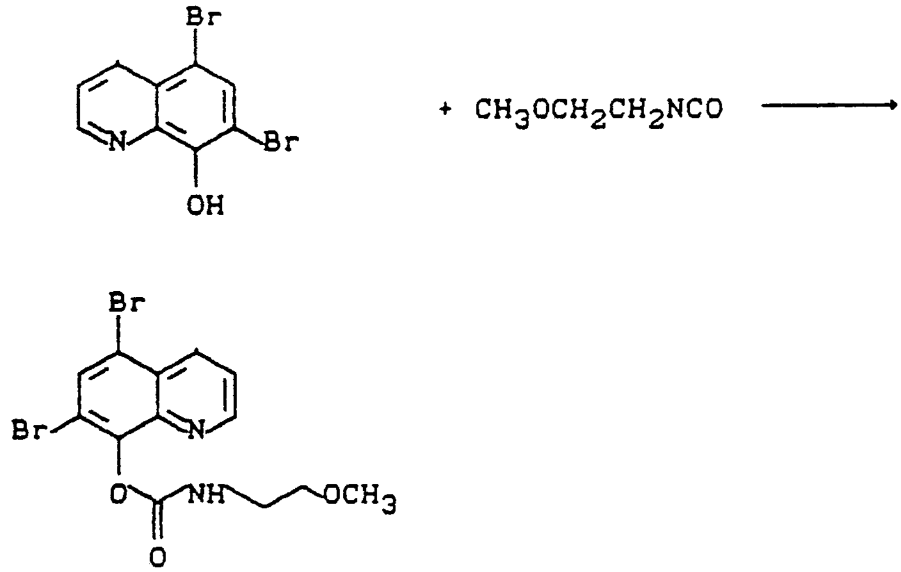


Verwendet man beispielsweise 2-Methyl-8-hydroxychinolin und N-Methyl-N-(3,6-dioxaheptyl)-carbamidsäurechlorid als Ausgangsstoffe, so kann der Reaktionsablauf der Verfahrensvarinate b) durch das folgende Formelschema wiedergegeben werden:

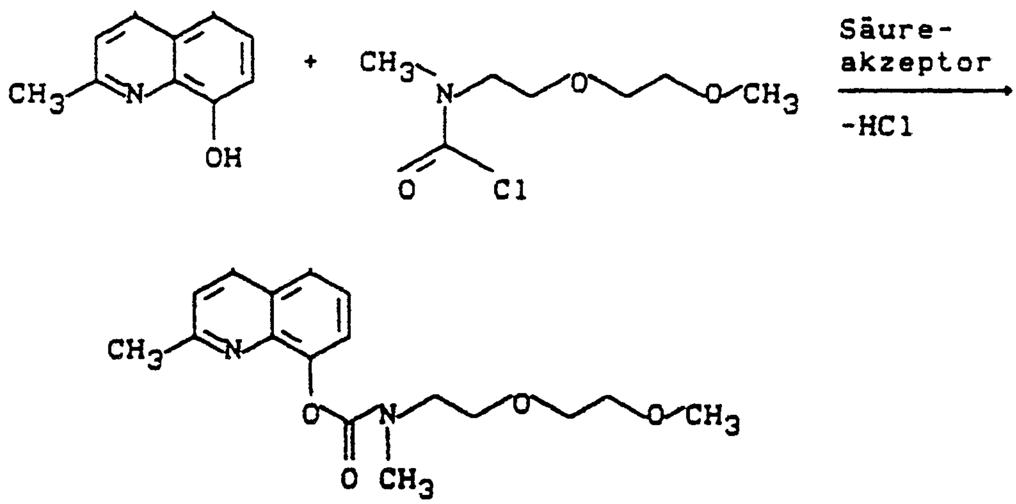


Die zur Durchführung der erfindungsgemäßen Verfahrensvarianten a) und b) als Ausgangsstoffe benötigten 8-Hydroxychinoline sind durch die Formel (II) allgemein definiert.

In dieser Formel (II) haben die Reste $R^1$, $R^2$ und $R^3$ die bereits im Zusammenhang mit der Beschreibung der erfindungsgemäßen Verbindungen der Formel (I) gegebenen Bedeutungen. Die Verbindungen sind größtenteils bekannt und nach Analogieverfahren herstellbar.

Die zur Durchführung der erfindungsgemäßen Verfahrensvariante a) außerdem benötigten Isocyanate sind durch die Formel (III) allgemein definiert. In dieser Formel (III) haben die Reste $R^5$ bis $R^{11}$ und n die bereits im Zusammenhang mit der Beschreibung der erfindungsgemäßen Verbindungen der Formel (I) gegebenen Bedeutung. Die Verbindungen sind bekannt und nach Analogieverfahren herstellbar (vgl. "Methoden der organischen Chemie", Houben-Weyl, Bd. E4, Kohlensäure-Derivate, Georg Thieme Verlag Stuttgart, New York, S. 738 ff (1983)).

Die weiterhin zur Durchführung der erfindungsgemäßen Verfahrensvariante b) benötigten Halogencarbonylverbindungen sind durch die Formel (IV) allgemein definiert. In dieser Formel (IV) haben die Reste Hal, $R^4$ bis $R^{11}$ und n die bereits im Zusammenhang mit der Beschreibung der erfindungsgemäßen Verbindungen der Formel (I) gegebenen Bedeutungen.

Die Verbindungen der Formel (IV) sind größtenteils bekannt oder können nach Analogieverfahren hergestellt werden [vgl. "Methoden der organischen Chemie" Houben-Weyl, Bd. E4, Kohlensäure-Derivate,

Georg Thieme Verlag Stuttgart, New York, S. 36 ff (1983)].

Als Verdünnungsmittel zur Durchführung der erfindungsgemäßen Verfahrensvarianten a) und b) kommen praktisch alle inerten organischen Verdünnungsmittel infrage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glykoldimethylether und Diglykoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z.B. Acetonitril und Propionitril, Amide, wie z.B. Dimethylformamid, Dimethylacetamid und N-Methylpyrrolidon, sowie Dimethylsulfoxid, Tetramethylensulfon und Hexamethylphosphorsäuretriamid.

Die erfindungsgemäßen Verfahrensvarianten a) und b) werden im allgemeinen bei Temperaturen zwischen -50° C und 120° C durchgeführt. Bevorzugt wird der Bereich zwischen 0° C und 110° C. Die Umsetzungen werden im allgemeinen bei Normaldruck durchgeführt.

Die Verfahrensvariante b) wird gegebenenfalls in Gegenwart von Säureakzeptoren durchgeführt. Als Säureakzeptoren können alle üblichen Säurebindemittel Verwendung finden. Besonders bewährt haben sich Alkalicarbonate und -alkoholate, wie Natrium- und Kaliumcarbonat, Natrium-und Kaliummethylat bzw. -ethylat, ferner aliphatische, aromatische oder heterocyclische Amine, beispielsweise Triethylamin, Trimethylamin, Dimethylanilin, 1,8-Diazabicyclo-(5,4,0)-undec-7-en, Dimethylbenzylamin und Pyridin.

Die Verfahrensvariante a) kann gegebenenfalls in Gegenwart von Basen durchgeführt werden. Als Basen können die bereits bei den Säureakzeptoren aufgeführten Basen Verwendung finden.

Zur Durchführung der erfindungsgemäßen Verfahrensvariante a) setzt man vorzugsweise auf ein Mol 8-Hydroxychinolin der allgemeinen Formel (II) 1 bis 2 Mol, insbesondere 1 bis 1,4 Mol, der Verbindungen der allgemeinen Formel (III) ein.

Zur Durchführung der erfindungsgemäßen Verfahrensvariante b) setzt man vorzugsweise auf 1 Mol 8-Hydroxychinolin der allgemeinen Formel (II) 1 bis 2 Mol, insbesondere 1 bis 1,4 Mol, der Verbindungen der Formel (IV) ein.

Die Aufarbeitung erfolgt nach üblichen Methoden, beispielsweise durch Extraktion der Produkte mit Toluol oder Methylenchlorid aus der mit Wasser verdünnten Reaktionsmischung, Waschen der organischen Phase mit Wasser, Trocknen und Destillieren oder sogenanntes "Andestillie ren", d.h. längeres Erhitzen unter vermindertem Druck auf mäßig erhöhte Temperaturen, um sie von den letzten flüchtigen Bestandteilen zu befreien, oder durch chromatographische Reinigung über Kieselgel oder z.B. durch Kristallisation. Zur Charakterisierung der Verbindungen dienen Brechungsindex, Schmelzpunkt, $R_f$-Wert oder Siedepunkt.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch insbesondere als Fungizide geeignet.

Fungizide Mittel im Pflanzenschutz werden z.B. eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz z.B. zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);

Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglykol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben,

Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Weiterhin können die erfindungsgemäßen Substanzen als Mikrobizide in mikrobiziden Mitteln zum Schutz technischer Materialien vor Schädigung oder Zerstörung durch Mikroorganismen verwendet werden. Technische Materialien sind stets dem Angriff einer großen Zahl verschiedener Mikrobenarten ausgesetzt, so daß ein zuverlässiger Schutz nur durch die Anwendung von Mikrobiziden mit breitem Wirkungsspektrum zu erzielen ist.

Die erfindungsgemäßen, sich vom 8-Oxy-chinolin ableitenden substituierten Carbamate stellen überraschenderweise bessere mikrobizide Materialschutzstoffe dar als 8-Oxy-chinolin selbst (s. Chemie der Pflanzenschutz- und Schädlingsbekämpfungsmittel, R. Wegler, Bd. 2, S. 112, Springer Verlag 1970), das aufgrund seiner geruchlichen und verfärbenden Eigenschaften als Materialschutzstoff viel zu wünschen übrig läßt. Die erfindungsgemäßen Substanzen sind hingegen fast farblos, z.T. weiß kristallin und nicht geruchsintensiv; sie eignen sich auch deswegen vorzüglich als Mikrobizide für den Schutz technischer Materialien.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz, dauerhaft verfärbende und holzzerstörende Pilze, sowie gegen Schleimorganismen und Algen.

Es seien beispielsweise Mikroorganismen der folgenden Gattungen genannt:

Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Je nach Anwendungsgebiet kann ein erfindungsgemäßer Wirkstoffe in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder

Methylethylketon, flüssige Kohlenwasserstoffe, wie Benzinfraktionen, halogenierte Kohlenwasserstoffe, wie 1,2-Dichlorethan, sein.

Mikrobizide Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen Wirkstoffe können auch in Mischung mit anderen bekannten Wirkstoffen vorliegen. Beispielsweise seien die folgenden Wirkstoffe genannt: Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Rhodanidomethylthiobenzthiazol, Organo-Zinnverbindungen, Methylenbisthiocyanat, Phenolderivate, wie 2-Phenylphenol, (2,2'-Dihydroxy-5,5'-dichlor)-diphenylmethan und 3-Methyl-4-chlor-phenol, Azolfungizide wie beispielsweise 1-Aryl-3-hydroxy-3-alkyl-4-(1,2,4-triazol-1-yl)butane.

Die Herstellung und die Verwendung der erfindungsgemäßen Wirkstoffe geht aus den nachfolgenden Beispielen hervor.

Herstellungsbeispiele

Beispiel 1

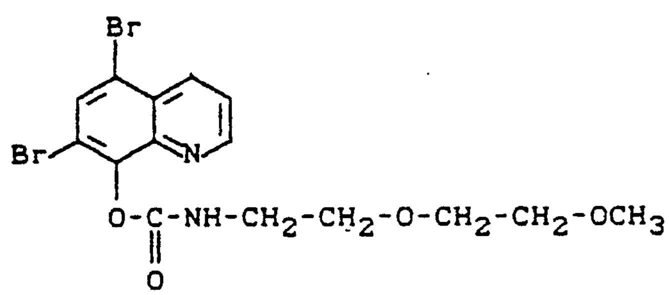

30,3 g (0,1 Mol) 5,7-Dibrom-8-hydroxychinolin werden in 50 ml Toluol gelöst und bei 20° C zunächst mit 17,4 g (0,11 Mol) Methoxyethoxyethylisocyanat, dann mit 30 mg (0,2 mMol) 1,8-Diaza-bicyclo-(5,4,0)-undec-7-en (DBU) versetzt. Man rührt 1 h bei 20° C und fügt dann erneut 2 g (12,6 mMol) Isocyanat zur Reaktionsmischung. Man erwärmt zunächst 1 h bei 50° C, dann 18 h bei 70° C, kühlt auf 0° C ab und saugt den ausgefallenen Feststoff ab. Anschließend wird aus einem Hexan/Toluolgemisch umkristallisiert.
Man erhält 40,2 g (90 % der Theorie) an 5,7-Dibrom-8-(3,6-dioxaheptylaminocarbonyl)-oxychinolin mit einem Schmelzpunkt von 104° C.

Beispiel 2

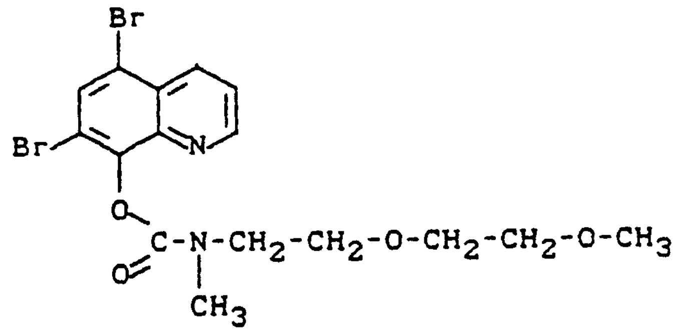

6,1 g (0,02 Mol) 5,7-Dibrom-8-hydroxychinolin, 3 g (0,022 Mol) Kaliumcarbonat, 4,3 g (0,022 Mol) Methyl-(3,6-dioxaheptyl)-carbamidsäurechlorid und 22 mg (0,2 mMol) 1,4-Diaza-bicyclo-[2,2,2]-octan (DABCO) werden in 50 ml N-Methylpyrrolidon suspendiert und einen Tag auf 100° C erhitzt.

Anschließend fügt man erneut 3,4 g Kaliumcarbonat hinzu und erwärmt weitere 18 h auf 140° C, gibt dann die Reaktionsmischung auf 200 ml Wasser, extrahiert mit Methylenchlorid und destilliert im Wasserstrahlvakuum das Lösungsmittel ab. Zur weiteren Reinigung wird über Kieselgel (Laufmittel : Petrolether : Aceton = 7:3) chromatographiert.

Man erhält 2,4 g (26 % der Theorie) an 5,7-Dibrom-8-(3,6-dioxaheptyl-N-methylaminocarbonyl)-oxychinolin mit einem Schmelzpunkt von 34° C.

In entsprechender Weise und gemäß den allgemeinen Angaben zur Herstellung erhält man die folgenden Oxychinolinderivate der Formel:

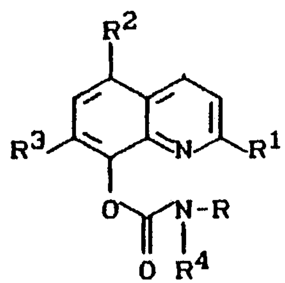

R = 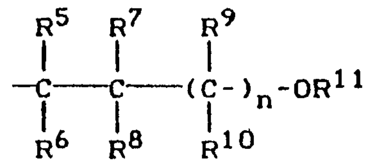

| Beispiel Nr. | $R^1$ | $R^2$ | $R^3$ | $R^4$ | R | phys. Daten |
|---|---|---|---|---|---|---|
| 3 | H | H | H | H | $-CH_2CH_2OCO$-t-Bu | Fp. 105 |
| 4 | H | H | H | H | $-CH_2C(CH_3)_2CH_2OCO$-t-Bu | Fp. 75 |
| 5 | H | Br | $-CH_2CHBrCH_2Br$ | H | $-(CH_2)_2OCH_3$ | Fp. 100 |
| 6 | H | H | H | H | $-(CH_2)_3OCOCH_3$ | Fp. 88 |
| 7 | H | Cl | H | H | $-(CH_2)_2OCH_3$ | Fp. 118-120 |
| 8 | H | Cl | Cl | H | $-(CH_2)_2OCH_3$ | Fp. 111 |
| 9 | H | Br | Br | H | $-(CH_2)_2OCH_3$ | Fp. 118 |
| 10 | H | Br | Br | H | $-(CH_2)_2O(CH_2)_2O-C_4H_9$-n | Fp. 50 |
| 11 | H | Br | Br | H | $-(CH_2)_2O(CH_2)_2OC_2H_5$ | Fp. 77 |
| 12 | H | Br | Br | H | $-(CH_2)_2OCO$-t-Bu | Fp. 120 |
| 13 | $-CH_3$ | H | H | H | $-(CH_2)_2O(CH_2)_2OCH_3$ | Fp. 84 |
| 14 | H | H | $-CH=CH-CH_3$ | H | $-(CH_2)_2OCH_3$ | Fp. 100 |
| 15 | H | Cl | H | H | $-(CH_2)_2O(CH_2)_2OCH_3$ | Fp. 61 |
| 16 | H | Cl | Cl | H | $-(CH_2)_2O(CH_2)_2OCH_3$ | Fp. 116 |
| 17 | H | Cl | $-CH_2CH=CH_2$ | H | $-(CH_2)_2O(CH_2)_2OCH_3$ | $n_D^{20}$ 1,5696 |

| Beispiel Nr. | $R^1$ | $R^2$ | $R^3$ | $R^4$ | R | phys. Daten |
|---|---|---|---|---|---|---|
| 18 | H | H | $-CH_2CH=CH-CH_3$ | H | $-(CH_2)_2O(CH_2)_2OCH_3$ | Fp. 85 |
| 19 | H | H | H | H | $-(CH_2)_2OCH_3$ | Fp. 95-101 |
| 20 | H | H | H | H | $-(CH_2)_2O(CH_2)_2OCH_3$ | Fp. 72 |
| 21 | H | H | H | H | $-(CH_2)_2O(CH_2)_2O$-Bu-n | $n_D^{20}$ 1,5349 |
| 22 | H | H | H | H | $-CH(CH_3)CH_2OCH_2$-t-Bu | Fp. 110 |
| 23 | H | H | H | H | $-CH(CH_3)CH_2O$-s-Bu | Fp. 84 |
| 24 | H | H | H | H | $-CH(CH_3)CH_2O$-i-Pr | Fp. 58 |
| 25 | H | H | H | H | $-CH(CH_3)CH_2O-C_3H_7$-n | Fp. 65 |
| 26 | H | H | H | H | $-CH(CH_3)CH_2OC_2H_5$ | Fp. 52 |
| 27 | H | H | H | H | $-CH(C_2H_5)CH_2OCH_3$ | Fp. 73 |
| 28 | H | H | H | H | $-CH(CH_3)CH_2OCH_3$ | Fp. 50 |
| 29 | H | H | H | H | $-CH(CH_3)CH_2OCOCH_3$ | Fp. 69 |
| 30 | H | Br | Br | H | $-CH_2CH(CH_3)OCOCH_3$ | Fp. 100 |
| 31 | H | Br | Br | H | $-CH_2CH(CH_3)OCO$-t-Bu | Fp. 87 |
| 32 | H | Br | Br | H | $-CH_2C(CH_3)_2CH_2OCOCH_3$ | Fp. 85 |
| 33 | H | Br | Br | H | $-CH_2C(CH_3)_2CH_2OCO$-t-Bu | Fp. 119 |
| 34 | H | H | H | H | $-CH_2CH(CH_3)OCO$-t-Bu | Fp. 106 |
| 35 | H | Br | Br | H | $-(CH_2)_3OCOCH_3$ | Fp. 106 |
| 36 | H | Br | Br | H | $-(CH_2)_3OCO$-t-Bu | Fp. 65 |
| 37 | H | H | H | H | $-(CH_2)_3OCO$-t-Bu | Fp. 73 |
| 38 | H | H | H | H | $-CH_2CH(CH_3)OCOCH_3$ | Fp. 85 |

Beispiel 39

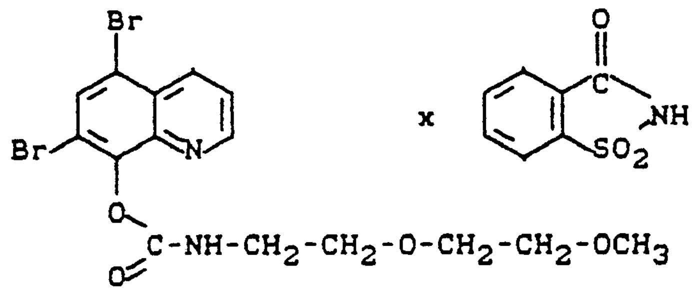

10 g (0,0223 Mol) 5,7-Dibrom-8-(3,6-dioxaheptylaminocarbonyl)-oxychinolin und 4,1 g (0,0223 Mol) Saccharin werden in 250 ml Tetrahydrofuran gelöst, 30 Minuten bei 20° C gerührt und anschließend durch Einrotieren im Wasserstrahlvakuum vom Lösungsmittel befreit.

Man erhält 14 g (quant.) an 5,7-Dibrom-8-(3,6-dioxaheptylaminocarbonyl)-oxychinolin-Saccharinsalz mit einem Schmelzpunkt von 145° C.

In analoger Weise werden die Additionssalze von 5,7-Dibrom-8-(3,6-dioxaheptylaminocarbonyl)-oxychinolin mit folgenden Carbonsäuren erhalten:

| Beispiel Nr. | add. Carbonsäure | phys. Daten |
|---|---|---|
| 40 | x $HOOC-CH_2-CH_2-COOH$ | Fp. 105° C |
| 41 | x $C_{17}H_{35}COOH$ | Fp. 100-102° C |

Herstellung der Ausgangsprodukte der Formel (III)

Beispiel III

1. 2-Methoxy-ethylisocyanat

1.1 Herstellung von N-(2-Methoxyethyl)-carbamid-säure-2-ethylhexyl)-ester (entsprechend der EP-PS 0 027 940)

In einem 4 l Rundkolben mit Rückflußkühler werden 225 g (3,0 mol) 2-Methoxyethylamin, 180 g (3,0 mol) Harnstoff, 1950 g (15 mol) 2-Ethylhexanol und 3 g Dibutylzinnoxid vorgelegt und unter Rühren erhitzt. Bei Temperaturen oberhalb 110° C setzt eine lebhafte Ammoniak-Entwicklung ein, Ammoniak-Gas wird über den Rückflußkühler abgeleitet und in Wasser absorbiert. Im Verlaufe von 3 Stunden wird die Temperatur auf 180° C (Rückfluß) erhöht, wobei die Ammoniak-Abspaltung praktisch zum Stillstand kam. Nach weiteren 2 Stunden am Rückfluß wird das Gemisch durch Spülen mit Stickstoff von Restgehalten an Ammoniak befreit und anschließend fraktioniert destilliert. Nach einem hauptsächlich aus Ethylhexanol bestehenden Vorlauf destilliert das gesuchte Urethan bei 98 - 100° C/0,1 mbar als farblose Flüssigkeit über (Ausbeute: 617 g = 89 % der Theorie). Als Destillationsrückstand bleiben 27 g braune Flüssigkeit zurück, die beim Abkühlen pastös erstarrt.

1.2 Thermische Spaltung von N-(2-Methoxyethyl)-carbamidsäure-(2-ethylhexyl)-ester (entsprechend der EP 0 054 817)

Als Spaltungsapparatur dient ein mit Tropftrichter und Rührer versehener Rundkolben, auf den zwei übereinander angeordnete Rückflußkühler aufgesetzt werden, die mittels Wärmeträgeröl thermostatisiert werden. Zwischen den beiden Rückflußkühlern ist ein Entnahmeboden ängebracht.

Das Urethan wird kontinuierlich ohne Rückstandsabtrennung gespalten.

Druck: 29 mbar
Temperatur: Spaltungskolben: 174-178° C
Ölvorlauf unterer Kühler: 135° C
Ölvorlauf oberer Kühler: 65° C
mittlere Verweilzeit: etwa 4 Stunden
Spaltungsfraktionen:
Entnahmeboden: 70,1 Gew.-% Ethylhexanol
Kopf des oberen Kühlers: 92,5 Gew.-% Isocyanat

Nach Beendigung der Spaltung werden die erhaltenen Gemische jeweils fraktioniert destilliert.

Gesamtergebnis der Spaltung:
Urethan: eingesetzt: 3,11 kg
zurückgewonnen: 0,84 kg
umgesetzt:2,27 kg
2-Methoxyethylisocyanat, destilliert: 920 g
(Kp. 124° C), Reinheit: 99,3 % (GC)
→ Selektivität: 92 %
2-Ethylhexanol: 1,23 kg
→ Selektivität: 96 %
Rückstand: 114 g

## Anwendungsbeispiele

### Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20° C und einer relativen Luftfeuchtigkeit von ca. 80 % aufgestellt, um die Entwicklung von Mehltaupusteln zu begünstigen.

7 Tage nach der Inokulation erfolgt die Auswertung.

Die Verbindungen der Herstellungsbeispiele (1) und (40) zeigen bei einer beispielhaften Wirkstoffkonzentration von 0,025 % einen Wirkungsgrad von 100 %.

### Beispiel B

Leptosphaeria nodorum-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20° C und 100 % relativer Luftfeuchtigkeit in einer Inkubationskabine.

Die Pflanzen werden in einem Gewächshaus bei einer Temperatur von ca. 15° C und einer relativen Luftfeuchtigkeit von ca. 80 % aufgestellt.

10 Tage nach der Inokulation erfolgt die Auswertung.

Die Verbindungen der Herstellungsbeispiele 1, 8, 9, 16, 19, 39, 40 und 41 zeigen bei einer beispielhaften Wirkstoffkonzentration von 0,025 % einen Wirkungsgrad von 80 bis 100 %.

## Beispiel C

Botrytis-Test (Bohne)/protektiv

Lösungsmittel: 4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20° C aufgestellt. 3 Tage nach der Inokulation wird die Größe der Befallsflecken auf den Blättern ausgewertet.

Die Verbindung des Herstellungsbeispiels 1 zeigt bei einer beispielhaften Wirkstoffkonzentration von 100 ppm einen Wirkungsgrad von 90 %.

## Beispiel D

Venturia-Test (Apfel) / protektiv

Lösungsmittel: 4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20° C und 100 % relativer Luftfeuchtigkeit in einer Inkubationskabine.

Die Pflanzen werden dann im Gewächshaus bei 20° C und einer relativen Luftfeuchtigkeit von ca. 70 % aufgestellt.

12 Tage nach der Inokulation erfolgt die Auswertung.

Die Verbindungen der Herstellungsbeispiele (1) und (10) zeigen bei einer beispielhaften Konzentration von 5 ppm einen Wirkungsgrad von nahezu 100 %.

## Beispiel E

Zum Nachweis der Wirksamkeit gegen Pilze werden die minimalen Hemm-Konzentrationen (MHK) von erfindungsgemäßen Wirkstoffen bestimmt:

Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2-wöchiger Lagerung bei 28° C und 60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt; sie ist in der nachstehenden Tabelle E angegeben.

Tabelle E

| MHK's in mg/l bei der Einwirkung erfindungsgemäßer Verbindungen auf Pilze | | | | | | | | | |
|---|---|---|---|---|---|---|---|---|---|
| Testorganismen | Verbindungen der Herstellungsbeispiele | | | | | | | | |
| | (19) | (14) | (20) | (1) | (15) | (16) | (17) | (18) | (21) |
| Alternaria tenuis | 20 | 35 | 35 | 20 | 10 | 20 | 75 | 50 | |
| Aspergillus niger | 100 | 20 | 200 | 50 | 50 | 50 | 100 | 50 | 200 |
| Aureobasidium pullulans | 50 | 50 | 20 | 20 | 20 | 10 | 100 | 50 | |
| Chaetomium globosum | 50 | 15 | 75 | 50 | 20 | 20 | 50 | 50 | 100 |
| Cladosporium cladosporioides | 50 | 10 | 50 | 50 | 20 | 20 | 50 | 50 | |
| Lentinus tigrinus | 50 | 20 | 50 | 50 | 20 | 20 | 75 | 50 | |
| Penicillium glaucum | 50 | 20 | 100 | | 50 | 50 | 100 | 75 | 100 |
| Polyporus versicolor | | | | | | | | | |
| Sclerophoma pityophila | 20 | 20 | 20 | 20 | 10 | 10 | 100 | 20 | |
| Trichoderma viride | 200 | 350 | 200 | | 50 | 50 | 200 | 500 | |

Beispiel F

Wirkung gegen Bakterien

Ein Agar, der als Nährmedium Bouillon enthält, wird mit erfindungsgemäßen Wirkstoffen in Konzentrationen von 1 bis 5.000 ppm versetzt. Darauf infiziert man das Nährmedium jeweils mit den in Tabelle II aufgeführten Testorganismen und hält das infizierte Medium 2 Wochen bei 28° C und 60 bis 70 % rel. Luftfeuchtigkeit. Die MHK ist die niedrigste Konzentration an Wirkstoff, bei der keinerlei Bewuchs durch die verwendete Mikrobenart erfolgt. Die MHK-Werte sind in Tabelle F wiedergegeben.

Tabelle F

| Angabe der MHK-Werte in mg/l bei der Einwirkung der unten angegebenen Wirkstoffe auf Bakterien. | | | | | | | | |
|---|---|---|---|---|---|---|---|---|
| Testorganismen | MHK in mg/l der Verbindungen der Herstellungsbeispiele | | | | | | | |
| | (19) | (20) | (1) | (15) | (16) | (17) | (18) | (21) |
| Escherichia coli | 75 | 100 | <20 | <20 | <20 | 50 | 50 | 100 |
| Staphylococcus aureus | 35 | 100 | <20 | <20 | <20 | <20 | 50 | 50 |

Beispiel G (Wirkung gegen Schleimorganismen)

Erfindungsgemäße Substanzen werden in Konzentrationen von jeweils 0,1 bis 100 mg/l in Allens Nährlösung (Arch. Mikrobiol. 17, 34 bis 53 (1952)), die in 4 l sterilem Wasser, 0,2 g Ammoniumchlorid, 4,0

g Natriumnitrat, 1,0 g Dikaliumhydrogenphosphat, 0,2 g Calciumchlorid, 2,05 g Magnesiumsulfat, 0,02 g Eisenchlorid und 1 % Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach 3-wöchiger Kultur bei Raumtemperatur noch völlig klar, d.h. die in wirkstofffreien Nährlösungen nach 3 bis 4 Tagen bemerkbare starke Vermehrung der Mikroben und Schleimbildung unterbleibt.

Tabelle G

| MHK-Werte in mg/l bei der Einwirkung der unten angegebenen Substanz auf Schleimoganismen | |
|---|---|
| Verbindungen der Herstellungsbeispiele | MHK in mg/l |
| (19) | >20 - <50 |
| (20) | >20 - <50 |
| (1) | ~20 |
| (15) | >20 - <50 |
| (16) | 10 - <20 |
| (17) | ≧20 - <50 |
| (18) | >20 - <50 |

<u>Beispiel H</u> (Wirkung gegen Algen)

Eine Mischkultur von Grün-, Blau-, Braun- und Kieselalgen (Stichococcus bacillaris Naegeli, Euglena gracilis Klebs, Chlorella pyrenoidosa Chick, Phormidium foveolarum Gomont, Oscillatoria geminata Meneghini und Phaeodactylum tricornutum Bohlin) wird unter Durchperlen von Luft in Allens Nährlösung (Arch. Mikrobiol. 17, 34 bis 53 (1952)), die auf 4 l steriles Wasser 0,2 g Ammoniumchlorid, 4,0 g Natriumnitrat, 1,0 g Dikaliumhydrogenphosphat, 0,2 g Calciumchlorid, 2,05 g Magnesiumsulfat und 0,02 g Eisenchlorid enthält, gegeben. Nach 2 Wochen ist die Nährlösung durch intensives Algenwachstum tief grün-blau gefärbt. Das Absterben der Algen nach Zugabe erfindungsgemäßer Wirkstoffe erkennt man an dem Entfärben der Nährlösung.

Tabelle H

| Algen-abtötende Konzentrationen [mg/l] der unten angegebenen Substanzen | |
|---|---|
| Verbindungen der Herstellungsbeispiele | abtötende Konzentration [mg/l] |
| (19) | ≧100 |
| (20) | ≧100 |
| (1) | ≧100 |
| (15) | ~100 |
| (16) | ≦100 |
| (17) | ~100 |
| (18) | ~100 |

## Ansprüche

1. 8-Carbamoyloxychinolin-Derivate der allgemeinen Formel (I)

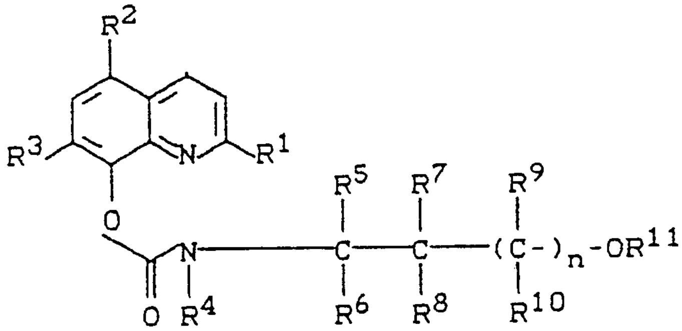

(I)

in welcher
$R^1$ für Wasserstoff oder Methyl steht und
$R^2$ und $R^3$ gleich oder verschieden sind und für Wasserstoff, Halogen, unsubstituiertes oder substituiertes Alkyl oder Alkenyl stehen und
n für null oder eins steht und
$R^{11}$ für unsubstituiertes oder substituiertes Alkyl, $COR^{12}$ oder einen Rest

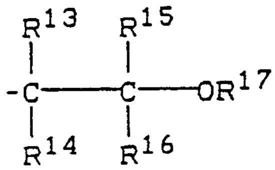

steht und
$R^4$ bis $R^{10}$ und $R^{13}$ bis $R^{16}$ gleich oder verschieden sind und für Wasserstoff oder unsubstituiertes oder substituiertes Alkyl stehen und
$R^{12}$ und $R^{17}$ für unsubstituiertes oder substituiertes Alkyl stehen, sowie deren Säureadditionssalze,
mit der Maßgabe, daß $R^{11}$ nicht für Alkyl oder einen Rest

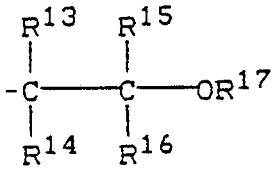

steht, wenn $R^1$ bis $R^4$ für Wasserstoff stehen und n für eins steht.

2. 8-Carbamoyloxychinolin-Derivate gemäß Anspruch 1, wobei in der Formel (I)
$R^1$ und $R^4$ für Wasserstoff oder Methyl stehen und
$R^2$ und $R^3$ gleich oder verschieden sind und für Wasserstoff, Halogen oder einen geradkettig oder verzweigten, unsubstituierten oder substituierten Alkyl- oder Alkenylrest mit 1 bis 6 bzw. 2 bis 6 Kohlenstoffatomen stehen und
n für null oder eins steht und
$R^{11}$ für geradkettig oder verzweigtes Alkyl mit 1 bis 6 Kohlenstoffatomen, $COR^{12}$ oder einen Rest

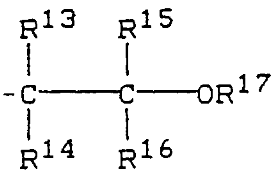

steht und

$R^5$ bis $R^{10}$ und $R^{13}$ bis $R^{16}$ gleich oder verschieden sind und für Wasserstoff oder geradkettig oder verzweigtes Alkyl mit 1 bis 6 Kohlenstoffatomen steht und

$R^{12}$ und $R^{17}$ für geradkettiges oder verzweigtes Alkyl mit 1 bis 6 Kohlenstoffatomen stehen, sowie deren Säureadditionssalze,

mit der Maßgabe, daß $R^{11}$ nicht für Alkyl oder einen Rest

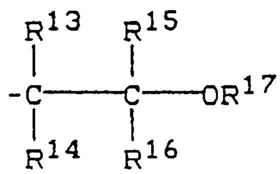

steht, wenn $R^1$ bis $R^4$ für Wasserstoff stehen und n für eins steht.

3. 8-Oxycarbamoyloxychinolin-Derivate gemäß Anspruch 1, wobei in der Formel (I)

$R^1$ und $R^4$ für Wasserstoff oder Methyl stehen und

$R^2$ und $R^3$ gleich oder verschieden sind und für Wasserstoff, Chlor, Brom oder Iod und geradkettig oder verzweigtes Alkyl oder Alkenyl mit 1 bis 4 bzw. 2 bis 4 Kohlenstoffatomen stehen und

n für eins steht und

$R^{11}$ für geradkettig oder verzweigtes Alkyl mit 1 bis 4 Kohlenstoffatomen, $COR^{12}$ oder einen Rest

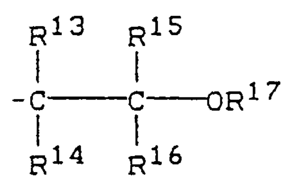

steht und

$R^5$ bis $R^{10}$ und $R^{13}$ bis $R^{16}$ gleich oder verschieden sind und für Wasserstoff oder geradkettig oder verzweigtes Alkyl mit 1 bis 4 Kohlenstoffatomen steht und

$R^{12}$ und $R^{17}$ für geradkettig oder verzweigtes Alkyl mit 1 bis 4 Kohlenstoffatomen stehen, sowie deren Säureadditionssalze,

mit der Maßgabe, daß $R^{11}$ nicht für Alkyl oder einen Rest

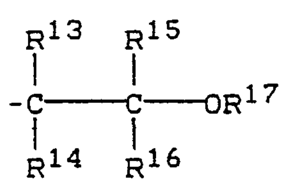

steht, wenn $R^1$ bis $R^4$ für Wasserstoff stehen und n für eins steht.

4. Verfahren zur Herstellung von 8-Carbamoyloxychinolin-Derivaten der allgemeinen Formel (I)

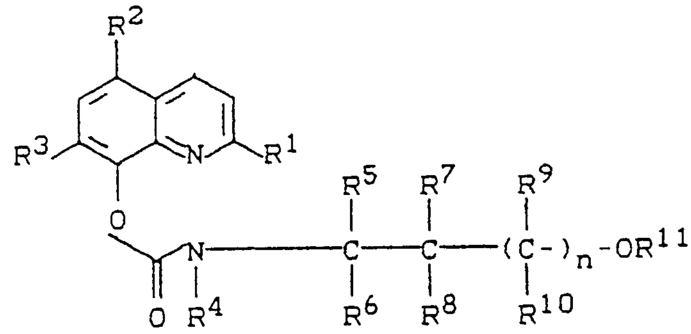

(I)

in welcher
$R^1$ für Wasserstoff oder Methyl steht und
$R^2$ und $R^3$ gleich oder verschieden sind und für Wasserstoff, Halogen, unsubstituiertes oder substituiertes Alkyl oder Alkenyl stehen und
n für null oder eins steht und
$R^{11}$ für unsubstituiertes oder substituiertes Alkyl, $COR^{12}$ oder einen Rest

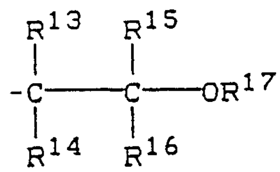

steht und
$R^4$ bis $R^{10}$ und $R^{13}$ bis $R^{16}$ gleich oder verschieden sind und für Wasserstoff oder unsubstituiertes oder substituiertes Alkyl stehen und
$R^{12}$ und $R^{17}$ für unsubstituiertes oder substituiertes Alkyl stehen,
mit der Maßgabe, daß $R^{11}$ nicht für Alkyl oder einen Rest

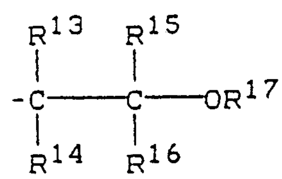

steht, wenn $R^1$ bis $R^4$ für Wasserstoff stehen und n für eins steht,
sowie deren Säureadditionssalze, dadurch gekennzeichnet, daß man 8-Hydroxychinoline der Formel (II)

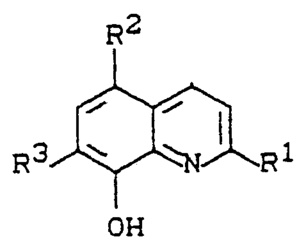

(II)

in welcher
$R^1$, $R^2$ und $R^3$ die obengenannten Bedeutungen haben,

a) mit Isocyanaten der Formel (III)

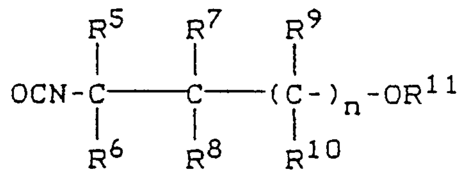 (III)

in welcher
$R^5$ bis $R^{11}$ und n die oben angegebenen Bedeutungen haben,
gegebenenfalls in Gegenwart einer Base und gegebenenfalls in Gegenwart von Verdünnungs-oder Lösungsmitteln umsetzt, oder

b) mit Halogencarbonylverbindungen der Formel (IV)

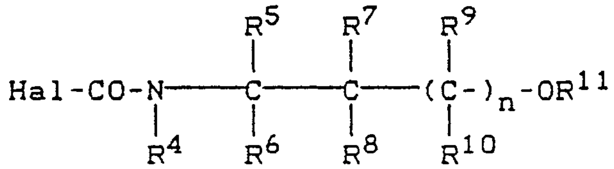 (IV)

in welcher
$R^4$ bis $R^{11}$ und n die oben angegebenen Bedeutungen haben, und
Hal für Halogen steht,
gegebenenfalls in Gegenwart eines Säureakzeptors und gegebenenfalls in Gegenwart eines Lösungs- oder Verdünnungsmittels umsetzt;
und gegebenenfalls anschließend eine Säure addiert.

5. Schädlingsbekämpfungsmittel, gekennzeichnet durch einen Gehalt an mindestens einem 8-Carbamoyloxychinolin-Derivat der Formel (I) nach den Ansprüchen 1 bis 7.

6. Verwendung von 8-Carbamoyloxychinolin-Derivaten der Formel (I) nach den Ansprüchen 1 bis 7 zur Bekämpfung von Schädlingen.

7. Mikrobizide Mittel zum Schutz technischer Materialien, gekennzeichnet durch einen Gehalt an mindestens einem 8-Carbamoyloxychinolin-Derivat der Formel (I) nach den Ansprüchen 1 bis 7.

8. Verwendung von 8-Carbamoyloxychinolin-Derivaten der Formel (I) nach den Ansprüchen 1 bis 7 zum Schutz von technischen Materialien vor Schädigung oder Zerstörung durch Mikroorganismen.

9. Verfahren zur Bekämpfung von Schädlingen und Mikroorganismen, dadurch gekennzeichnet, daß man 8-Carbamoyloxychinolin-Derivate der Formel (I) nach den Ansprüchen 1 bis 7 auf Schädlinge, Mikroorganismen und/oder ihren Lebensraum einwirken läßt.

10. Verfahren zur Herstellung von Schädlingsbekämpfungsmitteln und mikrobiziden Mitteln, dadurch gekennzeichnet, daß man 8-Carbamoyloxychinolin-Derivate der Formel (I) nach den Ansprüchen 1 bis 7 mit Streckmitteln und/oder oberflächenaktiven Mitteln vermischt.

Europäisches Patentamt

# EUROPÄISCHER RECHERCHENBERICHT

Nummer der Anmeldung

EP 90 10 1676

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
| D,A | DE-A-1 545 797 (BAYER AG)<br>* Seite 1, Seite 4, Verbindung VI *<br>--- | 1,5 | C 07 D 215/34<br>A 01 N 33/06 |
| D,A | DE-A-1 542 851 (CIBA-GEIGY AG)<br>* Seiten 1,2 *<br>--- | 1,5 | |
| A | DE-A-2 344 010 (ROUSSEL UCLAF PARIS)<br>* Seiten 1,2 *<br>--- | 1,5 | |
| A | DE-A-1 545 540 (AGRIPAT)<br>* Seite 1 *; & FR - A - 14 50570 (Kat. D)<br>----- | 1,5 | |
| | | | RECHERCHIERTE SACHGEBIETE (Int. Cl.5) |
| | | | C 07 D 215/00 |

Der vorliegende Recherchenbericht wurde für alle Patentansprüche erstellt

| Recherchenort | Abschlußdatum der Recherche | Prüfer |
|---|---|---|
| BERLIN | 01-06-1990 | KYRIAKAKOU G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)